# EUROPEAN PATENT APPLICATION

(11) **EP 2 833 148 A1**
(43) Date of publication of application: **04.02.2015**
(21) Application number: 13769634.0
(22) Date of filing: 29.03.2013
(51) Int. Cl.: G01N 33/569, G01N 33/53, C12Q 1/04, C12Q 1/68

(54) **METHOD FOR DETERMINING SEVERITY OF PNEUMOCOCCAL PNEUMONIA**

(30) Priority: 30.03.2012 JP 2012081038
(71) Applicant: Otsuka Pharmaceutical Co., Ltd., Chiyoda-ku Tokyo 101-8535 (JP)
(72) Inventor: FUKUSHIMA, Kiyoyasu, Nagasaki-shi Nagasaki 851-0113 (JP); TANAKA, Yumi, Tokyo 108-0075 (JP)
(74) Representative: Blodig, Wolfgang
(86) International application number: PCT/JP2013/059564
(87) International publication number: WO 2013/147173

(57) **Abstract**

Provided is a method that can more objectively and rapidly determine severity of pneumococcal pneumonia using blood of a subject. The method determines severity of pneumococcal pneumonia, the method including determining the severity based on the presence or absence of a pneumococcal antigen in blood collected from a subject and at least one biochemical value selected from a blood C-reactive protein (CRP) level, a white blood cell (WBC) count and a blood urea nitrogen (BUN) level.

## Description

### [Technical Field]

The present invention relates to a method for determining severity of pneumococcal pneumonia using blood of a subject.

### [Background Art]

Streptococcus pneumoniae (S. pneumoniae) is a bacterium that is most frequently detected as a causative bacterium for community-acquired pneumonia or lower respiratory tract infection and is one of causative bacteria associated with high morbidity and mortality not only in Japan but also in the whole world. Pneumococcal infections show high frequency of occurrence and are also apt to increase in severity. Accordingly, it is important to determine the causative bacteria as early as possible and to select a correct therapeutic drug from the viewpoints of improvement in prognosis, reduction in medical care cost, and prevention of the emergence of resistant bacteria.

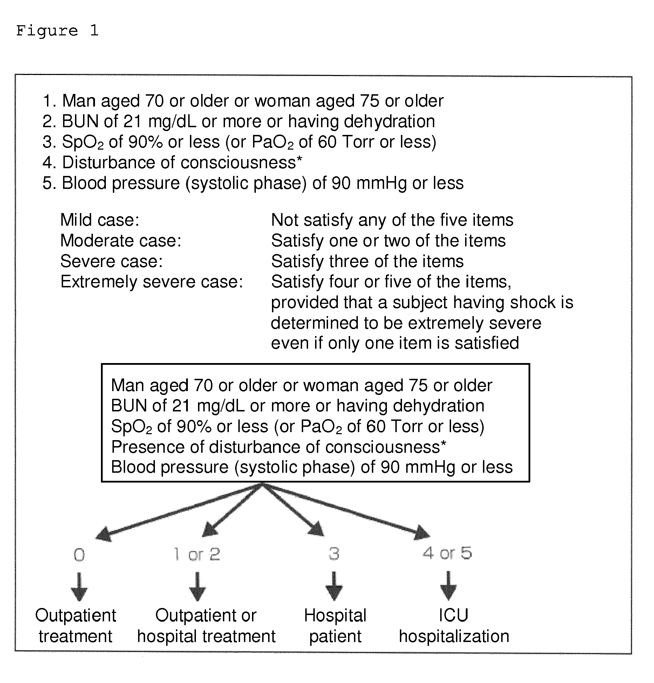

In general, pneumonia is comprehensively diagnosed based on, for example, clinical manifestations, physical findings, general examination findings, and chest radiograph. A patient diagnosed with pneumonia is then subjected to determination of severity in accordance with the A-DROP system shown in "Guidelines for the Management of Community-Acquired Pneumonia in Adults" (Non Patent Document 1). Based on the result, treatment, i.e., outpatient treatment, hospital treatment, or ICU treatment is selected. Here, the A-DROP system determines the severity based on the number of indices that apply to the subject among the following five indices: 1) men aged 70 or older or women aged 75 or older, 2) BUN of 21 mg/dL or more or having dehydration, 3) SpO₂ of 90% or less (or PaO₂ of 60 Torr or less), 4) presence of disturbance of consciousness, and 5) blood pressure (systolic phase) of 90 mmHg or less (see Figure 1).

Subsequently, an examination for identifying pathogenic bacteria are carried out, and a course of treatment is established depending on the detected pathogenic bacteria. However, the identification of pathogenic bacteria and the drug susceptibility test by culture tests take several days to obtain the results. Recently, a rapid test using a diagnostic agent employing a specific antibody against a pneumococcal antigen in, for example, sputum, epipharynx swab, or urine (e.g., "RAPIRUN (registered trademark) S. pneumoniae" (Otsuka Pharmaceutical Co., Ltd.), "RAPIRUN (registered trademark) S. pneumoniae HS (otitis media/sinusitis)" (Otsuka Pharmaceutical Co., Ltd.), or "BinaxNOW (registered trademark) Streptococcus pneumoniae" (Alere Medical Co., Ltd.)) is performed at an early stage, and a treatment plan is established at an early point.

It has been reported that bacteremia is observed in 10% of pneumonia patients as complications and that particularly the frequency reaches 60% or more in S. pneumoniae infection. Accordingly, it is important to detect bacteria present in blood. Examples of the method for detecting bacteria in blood include blood culture, RT-PCR, and antigen test. Blood culture is implemented in pneumonia as much as possible. However, blood culture takes time to obtain the test results, and its detection rate is low, about 20%, compared to that of RT-PCR. Accordingly, the method is not effective in urgent situations. RT-PCR has a high detection rate, but it needs a special apparatus and is expensive. Consequently, RT-PCR has not been widely spread in clinical practice. Regarding antigen test, in principle, there are reports on detection of, for example, a C-polysaccharide antigen (C-ps) in serum by, for example, ELISA (Non Patent Documents 2 and 3), but they are detection methods requiring pretreatment or lacking promptness as in ELISA. Consequently, the actual state is that antigen test has not been put to practical use yet.

Accordingly, a means that can objectively and rapidly determine severity of pneumonia, which is currently judged based on clinical manifestations and information of biochemical markers only, and also can estimate the pathogenic bacteria has been desired.

### [Citation List]

### [Non Patent Document]

[Non Patent Document 1] The JRS Guidelines for the Management of Community-Acquired Pneumonia in Adults, The Japanese Respiratory Society, 2007, pp. 9-12
[Non Patent Document 2] Gillespie S.H., et al., 1995, J. Clin. Pathol., 48: 803-806
[Non Patent Document 3] Coonrod J.D., et al., 1973, J. Lab. Clin. Med., 81: 778-786

### [Summary of the Invention]

### [Problems to be Solved by the Invention]

The present invention provides a method capable of more objectively and rapidly determining severity of pneumococcal pneumonia using blood of a subject.

### [Means for Solving the Problems]

The present inventors have investigated methods that can simply and objectively determine severity of pneumococcal pneumonia at the time of initial diagnosis, and have found that when a pneumococcal antigen in blood of a patient is measured and the resulting information is combined with a measured value of a specific biochemical marker (biochemical value), patients with pneumococcal pneumonia that would be determined to be severe or extremely severe by an A-DROP system can be satisfactorily distinguished from patients with pneumococcal pneumonia that would be determined to be mild or moderate by the A-DROP system to thereby detect severe pneumococcal pneumonia. In addition, since this method measures a pneumococcal antigen in blood, pneumococcal bacteremia can also be simultaneously determined.

The present invention relates to the following aspects 1) to 4):
1) A method for determining severity of pneumococcal pneumonia, comprising determining the severity based on the presence or absence of a pneumococcal antigen in blood collected from a subject and at least one biochemical value selected from a blood C-reactive protein (CRP) level, a white blood cell (WBC) count and a blood urea nitrogen (BUN) level;
2) The method according to aspect 1), wherein the pneumococcal pneumonia is classified as severe or extremely severe when the pneumococcal antigen is positive and the biochemical value is in the following range:
   CRP: 10 mg/dL or more,
   WBC: 10,000 cells/mm³ or less, or
   BUN: 20 mg/dL or more;
3) The method according to aspect 1) or 2), wherein the pneumococcal antigen is at least one selected from a C-polysaccharide antigen, a capsular antigen, and a cell membrane polysaccharide antigen; and
4) The method according to any one of aspects 1) to 3), wherein pneumococcal bacteremia is simultaneously evaluated.

### [Effects of the Invention]

The method of the present invention can objectively and rapidly judge severity of pneumonia, which have been judged based on clinical manifestations and information of biochemical markers only, and also can decide the place for treatment (outpatient treatment, hospital treatment, or ICU treatment) based on the severity, including estimation of the pathogenic bacteria.

Consequently, a suitable antibacterial agent can be selected at an early point, which leads to an improvement in prognosis, a reduction in medical care cost, and a prevention of the emergence of resistant bacteria.

In addition, the method of the present invention can detect a pneumococcal antigen in blood with a sensitivity equivalent to that of RT-PCR and therefore can simultaneously detrmine pneumococcal bacteremia.

### [Brief Description of the Drawings]

[Figure 1] Figure 1 shows a relationship between severity classification by A-DROP system and place for treatment.
[Figure 2] Figure 2 includes a graph and a table showing the line intensity (pneumococcal antigen detection sensitivity) at each serum dilution rate.
[Figure 3] Figure 3 is a diagram showing the line intensity (pneumococcal antigen detection sensitivity) in each blood sampling method.
[Figure 4] Figure 4 is a graph showing a correlation of a combination of the detection of blood pneumococcal antigen and CRP with severity.
[Figure 5] Figure 5 is a graph showing a correlation of a combination of the detection of blood pneumococcal antigen and WBC with severity.
[Figure 6] Figure 6 is a graph showing a correlation of a combination of the detection of blood pneumococcal antigen and BUN with severity.
[Figure 7] Figure 7 is a graph showing a correlation of a combination of the detection of blood pneumococcal antigen and neutrophils with severity.
[Figure 8] Figure 8 is a graph showing a correlation of a combination of the detection of blood pneumococcal antigen and Na with severity.
[Figure 9] Figure 9 is a graph showing a correlation of a combination of the detection of blood pneumococcal antigen and Cr with severity.
[Figure 10] Figure 10 is a graph showing a correlation of a combination of the detection of blood pneumococcal antigen and BNP with severity.

### [Modes for Carrying out the Invention]

Examples of the subject to which the method of the present invention is applied include patients with pneumococcal pneumonia, patients with suspected pneumococcal pneumonia, patients with suspected pneumococcal respiratory tract infection, patients with suspected systemic pneumococcal infection, patients with suspected pneumococcal inflammation, and healthy subjects who are suspected to have any of pneumococcal diseases represented by the above diseases. The patients mentioned here are not limited to human, and mammals other than human can be subjects of the present invention.

The "blood" collected from a subject encompasses blood (whole blood), plasma, and serum.

In measurement of a pneumococcal antigen, serum or plasma is preferably used, and 1- to 10-fold, preferably 2-fold diluted serum or plasma is preferred from the viewpoint of increasing the detection sensitivity.

Examples of the pneumococcal antigen used in the method of the present invention include a cell wall antigen (C-polysaccharide antigen (C-polysaccharide: C-ps)), a capsular antigen (capsular polysaccharide), and a cell membrane polysaccharide antigen (F-antigen: lipoteichoic acid/teichoic acid). Among these antigens, the C-ps, the capsular antigen, and the cell membrane polysaccharide antigen are preferred, and the C-ps, the capsular antigen, and the cell membrane polysaccharide antigen are more preferably measured or detected together.

In the method of the present invention, the presence or absence of the pneumococcal antigen in blood is measured, and the measurement encompasses quantitative and non-quantitative measurements. Examples of the non-quantitative measurement include mere measurement of whether or not a pneumococcal antigen is present, measurement of whether or not a certain amount or more of a pneumococcal antigen is present, and measurement of comparing the amount of a pneumococcal antigen to that of another sample (e.g., control sample). Examples of the quantitative measurement include measurement of the concentration of a pneumococcal antigen and measurement of the amount of a pneumococcal antigen.

In the present invention, that a pneumococcal antigen is positive means that the pneumococcal antigen is present at least in blood.

Preferred examples of the measurement method of a pneumococcal antigen include immunoassay using an antibody against the pneumococcal antigen. The antibody against the pneumococcal antigen may be any antibody that specifically binds to the pneumococcal antigen, and no limitation is imposed on the source, type (monoclonal or polyclonal) and shape of the antibody. Specifically, a known antibody such as a mouse antibody, a rat antibody, a human antibody, a chimeric antibody, or a humanized antibody can be used.

Examples of monoclonal antibodies derived from mammals include monoclonal antibodies produced by hybridomas and monoclonal antibodies produced by a host transformed with expression vectors containing antibody genes by genetic engineering. The monoclonal antibody-producing hybridoma can be basically produced through a known technique by performing immunization with a pneumococcal antigen as a sensitized antigen in accordance with a usual immunization method, fusing the resulting immune cells with known parent cells in accordance with a usual cell fusion method, and screening for monoclonal antibody-producing cells by a usual screening method.

The antibody may be a low-molecular antibody such as an antibody fragment or a modified antibody as long as having the characteristic of recognizing a pneumococcal antigen. Specific examples of the antibody fragment include Fab, Fab', F(ab')2, Fv, and diabody.

Specific examples of the antibody against a pneumococcal antigen include antibodies against C-ps, such as those described in 1) Nielsen, et al., Antibodies against pneumococcal C-polysaccharide are not protective, Microbial Pathogenesis 14: 299-305 (1993), 2) Gillespie, et al., Detection of C-polysaccharide in serum of patients with Streptococcus pneumoniae bacteremia, J. Clin. Pathol., 48: 803-806 (1995), 3) United States Patent No. 6824997 (1997), or 4) Ehara, et al., A novel method for rapid detection of Streptococcus pneumoniae antigen in sputum and its application in adult respiratory tract infection, Journal of Medical Microbiolog, 57: 820-82 (2008); antibodies against a capsular antigen, such as those described in 1) Coonrod, et al., Detection of type-specific pneumococcal antigens by counterimmunoelectrophoresis. 11. Etiologic diagnosis of pneumococcal pneumonia, J. Lab. Clin. Med., 81, 778-786 (1973), 2) Feigin, et al., Countercurrent immunoelectrophoresis of urine as well as of CSF and blood for diagnosis of bacterial meningitis, The Journal of Pediatrics, 89, 773-775 (1976), 3) Ajello, et al., Commercial Latex Agglutination Tests for Detection of Haemophilus influenza Type b and Streptococcus pneumoniae Antigens in Patients with Bacteremic Pneumonia, JOURNAL OF CLINICAL MICROBIOLOGY, 25(8), 1388-1391 (1987), 4) Ballaed, et al., Comparison of three latex agglutination kits and counterimmunoelectrophoresis for the detection of bacterial antigens in a pediatric population, Pediatr. Infect. Dis. J., 6(7), 630-634 (1987), or 5) Kobayashi, et al., Evaluation of Streptococcus Pneumoniae-urinary Antigen Detection kit in Patients with Community Acquired Pneumonia, The Journal of the Japanese Association for Infectious Diseases, Vol. 76, No. 12, 995-1002 (2002); and antibodies against a cell membrane polysaccharide antigen, such as those described in Hotomi, et al., Evaluation of a Rapid Immunochromatographic ODK-0901 Test for Detection of Pneumococcal Antigen in Middle Ear Fluids and Nasopharyngeal Secretions, PLoS one, 7(3), e33620 (2012).

The immunoassay using an antibody against a pneumococcal antigen can be any immunoassay known in the art, and examples thereof include radioimmunoassay (RIA), enzyme immunoassay (EIA) such as ELISA, latex turbidimetric immunoassay (LTIA), and immunochromatography. From the viewpoint of improving detection sensitivity, a sandwich method is preferred. From the viewpoint of simplicity and rapidness, immunochromatography is preferred. The immunochromatography allows diagnosis at bedside or diagnosis within a short time at the time of visit of a patient.

The label used in the immunoassay may be any label used in the art, and examples thereof include enzymes such as horseradish peroxidase (HRP), alkaline phosphatase, and β-galactosidase; radioisotopes (RI) such as 1251, 32P, 14C, 35S, and 3H; fluorescent materials such as FITC and tetramethyl rhodamine isocyanate; luminescent materials such as chemiluminescent materials; and visualized materials such as gold colloid and colored latex particles. In addition, there may be used a sensitization system using biotin in primary labeling and then avidin labeled with any of the above-mentioned labels or a detection process using a low-molecular material, such as digoxigenin, in primary labeling and then a material, such as an antibody, having affinity to the low-molecular material and labeled with any of the above-mentioned labels.

The measurement of a pneumococcal antigen in the present invention can also be performed using a commercially available S. pneumoniae detection kit such as "RAPIRUN S. pneumoniae HS (otitis media/sinusitis)" (Otsuka Pharmaceutical Co., Ltd.) and "BinaxNOW Streptococcus pneumoniae" (Alere Medical Co., Ltd.).

The biochemical value used in the present invention is one or more selected from a blood C-reactive protein (CRP) level, a white blood cell (WBC) count, and a blood urea nitrogen (BUN) level.

### (1) C-reactive protein (CRP)

CRP is a protein that appears in blood when an inflammatory reaction or tissue destruction is occurring in the body and has a property of binding to the C-polysaccharide of S. pneumoniae. CRP is generally used as an inflammatory marker.

CRP was defined as a parameter for determining severity of pneumonia in "Basic concept for the management of community-acquired pneumonia in adults", which is former guidelines established in 2000. However, in verification in "Guidelines for respiratory infection" established in 2007, there was a report on that CRP does not accurately reflect severity. Accordingly, in new guidelines, CRP was deleted from the items for determining severity (see Non Patent Document 1).

However, when used in combination with detection (positive) of a pneumococcal antigen, CRP can determine severe and extremely severe pneumococcal pneumonia (see Example 3). In this case, the cut-off value of CRP is 10 to 20 mg/dL.

That is, in the present invention, the CRP as an index of severe and extremely severe pneumococcal pneumonia is 10 mg/dL or more and preferably 20 mg/dL.

In the present invention, CRP measurement can be carried out by, for example, but not limited to, latex immunoturbidimetric assay.

### (2) White blood cell (WBC) count

It is known that white blood cell (WBC) count in blood generally increases in bacterial infection, but its level in blood decreases in severe pneumonia due to localization thereof in the lung. WBC was, as in CRP, defined as a parameter for determining severity of pneumonia in the past, but was deleted from the items for determining severity in new guidelines established in 2007 (see Non Patent Document 1).

However, when used in combination with detection (positive) of a pneumococcal antigen, WBC also can determine severe and extremely severe pneumococcal pneumonia (see Example 3). In this case, the cut-off value of WBC is 6,000 to 10,000 cells/mm³.

That is, in the present invention, the WBC as an index of severe and extremely severe pneumococcal pneumonia is within a distribution range of healthy subjects, or in the case of less than the range, for example, the WBC is 10,000 cells/mm³ or less and preferably 6,000 cells/mm³ or less.

In the present invention, WBC measurement can be measured with, for example, but not limited to, an automatic analyzer that can count blood cells.

### (3) Blood urea nitrogen (BUN) level

BUN represents the amount of nitrogen derived from urea in blood and is used as a kidney function marker.

BUN is known to be high in severe pneumonia patients showing highly enhanced dehydration and catabolism. When used in combination with detection (positive) of a pneumococcal antigen, BUN can determine severe and extremely severe pneumococcal pneumonia with higher accuracy (see Example 3). In this case, the cut-off value of BUN is, for example, about 20 mg/dL.

That is, in the present invention, the BUN as an index of severe and extremely severe pneumococcal pneumonia is, for example, 20 mg/dL or more.

In the present invention, BUN can be measured by, for example, but not limited to, a urease GLDH method or a urease ICDH method. Of these, the urease GLDH method is preferably used.

The method of the present invention measures a pneumococcal antigen in blood and therefore can simultaneously determine pneumococcal bacteremia. Here, the term "Pneumococcal bacteremia" means that S. pneumoniae is present in blood. That is, in the method of the present invention, that a blood pneumococcal antigen is positive suggests pneumococcal bacteremia. Although pneumococcal bacteremia has been conventionally determined by detecting S. pneumoniae in blood culture, the detection rate in blood culture is recognized to be low for not only S. pneumoniae but also microorganisms. Accordingly, in recent years, detection by RT-PCR is increasingly used (Resti, et al., Community-Acquired Bacteremic Pneumococcal Pneumonia in Children: Diagnosis and Serotyping by Real-Time Polymerase Chain Reaction Using Blood Samples, Clinical Infectious Diseases, 51(9): 1042-1049 (2010)). The method of the present invention can infer pneumococcal bacteremia in a simple manner without using RT-PCR.

### [Examples]

### <Material and method>

Serum or plasma was separated from whole blood collected from each patient who gave informed consent by usual centrifugation or precipitation method and was stored at -80°C.

### (1) Preparation of specimen

A serum fraction was obtained by collecting blood in a blood-collecting tube containing a serum separator and centrifuging the blood. Plasma was obtained by collecting blood in a blood-collecting tube containing EDTA-2Na or heparin and collecting the supernatant after centrifugation or standing. Both specimens were stored at -80°C.

### (2) Evaluation of optimal dilution rates of serum/plasma for measuring pneumococcal antigen

The optimal dilution rates of serum/plasma were evaluated with "RAPIRUN S. pneumoniae HS (otitis media/sinusitis)" (Otsuka Pharmaceutical Co., Ltd.). Serum/plasma was serially diluted with the specimen extraction reagent included in the kit, and a dilution rate providing the strongest test line intensity was determined. The line after measurement was photographed with a commercially available digital camera and was then evaluated for the intensity with a densitometer (ATTO Co., Ltd.).

### (3) Measurement of pneumococcal antigen

The presence or absence of a pneumococcal antigen in blood was measured with commercially available kits shown below.

### 1) RAPIRUN S. pneumoniae HS (otitis media/sinusitis) (Otsuka Pharmaceutical Co., Ltd.)

Two-fold diluted serum/plasma, which had been judged as an optimal dilution rate in the above (2), was dispensed in an amount of 150 µL into a sample cup included in the kit. The sample applying portion of a test stick (the reagent itself) was immersed in the 2-fold diluted serum/plasma, and the subsequent measuring procedure was performed in accordance with the package insert.

### 2) "BinaxNOW (R) Streptococcus pneumoniae" (Alere Medical Co., Ltd.)

The cotton swab included in the kit was immersed in the 2-fold diluted serum/plasma prepared in the above 1), and evaluation was performed in accordance with the package insert.

### (4) Other measurement items

### 1) Biochemical marker

CRP, WBC, neutrophil, BUN, Na, Cr, and BNP of the serum specimen were measured with a Hitachi automated analyzer LABOSPECT 008.

### 2) Culture

In order to estimate the pathogenic bacteria, blood and sputum were cultured. Each specimen was cultured in accordance with the standard of Clinical and Laboratory Standards Institute (CLSI).

### 3) PCR

S. pneumoniae PspA DNA in serum was quantitatively measured using the PspA gene of S. pneumoniae as a target in accordance with the method in a previous report (Ehara N., et al., 2008, Journal of Medical Microbiology, 57: 820-826). The detection sensitivity in the facility was 200 copies/µg DNA.

### Example 1

### (1) Severity determination

Biochemical markers and a pneumococcal antigen in blood were evaluated for the cases shown below that had been subjected to the severity determination using the A-DROP system described in the Guidelines for the Management of Community-Acquired Pneumonia in Adults (Non Patent Document 1). The number of cases of each severity is shown in Table 1.

**[Table 1]**

| | Cases other than pneumonia* | Pneumonia | | | |
|---|---|---|---|---|---|
| | | Mild | Moderate | Severe | Extremely severe |
| Number of specimens | 6 | 16 | 3 | 4 | 3 |

| | | | | | |
|---|---|---|---|---|---|
| *: Nontuberculous mycobacterial infection, bronchitis, bronchial asthma, pulmonary emphysema, and dehydration | | | | | |

### (2) Adjustment of optimal specimen concentration

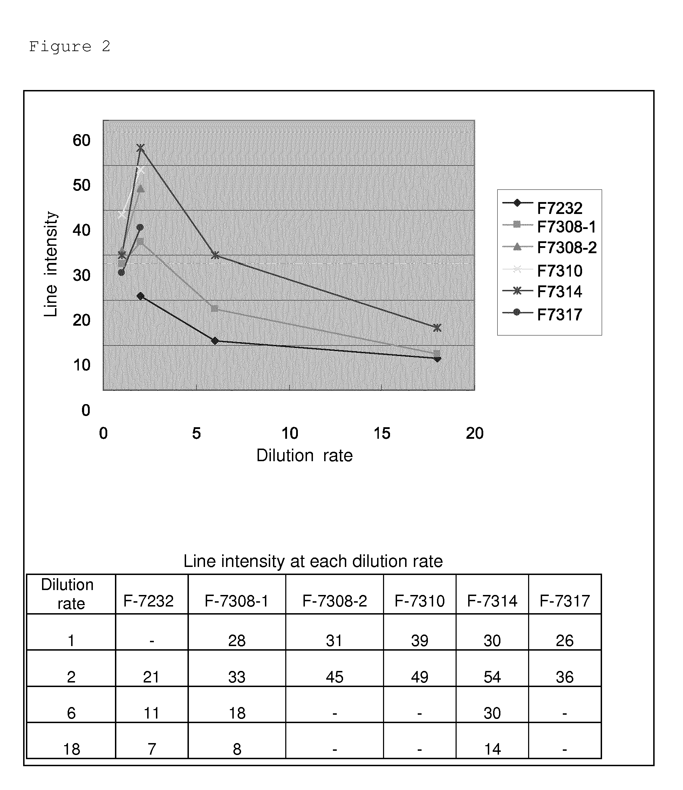

Different six serum samples were each measured with a kit "RAPIRUN S. pneumoniae HS (otitis media/sinusitis) " as an index before (dilution rate: 1-fold) and after 2-, 6-, and 18-fold dilution with the specimen extraction reagent included in the kit. The line intensities were compared. Figure 2 shows the line intensity at each dilution rate.

Figure 2 demonstrates that the line intensity showed a maximum value at 2-fold dilution in every serum specimen. The undiluted serum had high viscosity and tended to have a slow flow rate, and thus 2-fold dilution (undiluted serum : specimen extraction solution = 1:1) was believed to be the optimal specimen concentration in both promptness and line intensity.

### (3) Difference between blood sampling methods (specimen type)

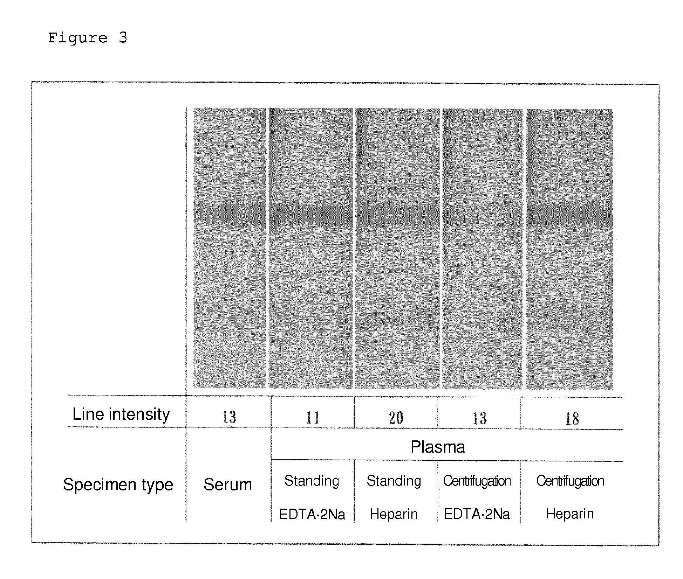

Serum and plasma were prepared from blood collected from one patient, wherein the plasma was prepared by four different procedures shown in Figure 3. The line intensities of the serum and plasma were evaluated with "RAPIRUN S. pneumoniae HS (otitis media/sinusitis)". Figure 3 also shows the results.

In every blood sampling method (serum, EDTA-2Na plasma, or heparin plasma) and in every separation method (centrifugation or standing separation), the line intensities were substantially the same. Thus, the line intensity was believed not to be affected by the blood sampling method and the separation method. Accordingly, in the subsequent evaluation, serum prepared by centrifugation was used.

### (4) Sensitivity and specificity of detection of blood pneumococcal antigen in each patient

The sensitivity and specificity of detection of blood pneumococcal antigen in each patient were calculated based on the results of a sputum culture test. The sensitivity is the ratio of the number of cases determined to be positive in each measurement method to the number of sputum culture positive cases. The specificity is the ratio of the number of cases determined to be negative in each measurement method to the number of sputum culture negative cases (Table 2).

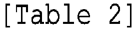

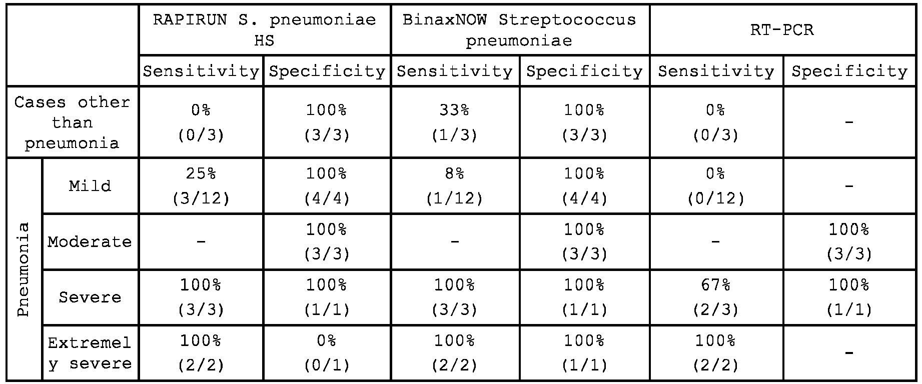

"RAPIRUN S. pneumoniae HS" and "BinaxNOW Streptococcus pneumoniae" both tended to increase the sensitivity as the severity of pneumonia increases, and the detection rates thereof were equivalent to that of "RT-PCR", which is known as a method of detecting S. pneumoniae with high sensitivity.

### Example 2: Results of analysis of severe or extremely severe cases

The blood pneumococcal antigen of the pneumonia cases determined to be severe or extremely severe was measured with "RAPIRUN S. pneumoniae HS" and "BinaxNOW Streptococcus pneumoniae", and the results were compared to those of blood PCR. As a result, five severe or extremely severe cases in which S. pneumoniae was detected in sputum culture were all positive in both kits. In PCR, four cases, excluding one case, were positive.

**[Table 3]**

| Specimen No. | Severity of pneumonia | Sputum | Urine | Blood | | | |
|---|---|---|---|---|---|---|---|
| | | Culture (S. pneumoniae) | Binax | Culture | PCR | Binax | RAPIRUN HS |
| F-7232 | Severe | positive | (+) | negative | 2×10⁴ | (+) | (+) |
| F-7481 | Severe | positive | (+) | negative | <2×10² | (+) | (+) |
| F-7164 | Severe | positive | (+) | NT | 2×10³ | (+) | (+) |
| F-7506 | Extremely severe | positive | (+) | negative | 1×10³ | (+) | (+) |
| F-7322 | Extremely severe* | positive | NT | NT | 6×10³ | (+) | (+) |
| F-7210 | Severe | negative | negative | negative | <2×10² | (-) | (-) |
| F-7550 | Extremely severe | negative | negative | negative | NT | (-) | (+) |

| | | | | | | | |
|---|---|---|---|---|---|---|---|
| *: The severity was unclear on the measurement day, but the severity was determined to be extremely severe 2 days after. NT: not tested RAPIRUN HS: RAPIRUN S. pneumoniae HS, Binax: BinaxNOW Streptococcus pneumoniae | | | | | | | |

### Example 3: Clinical course

Clinical course of one patient was evaluated. PCR, "RAPIRUN S. pneumoniae HS", and "BinaxNOW Streptococcus pneumoniae" were all negative on the first day of sampling specimens, but all turned to positive 2 days after. Thus, it was judged that the detection efficiencies of PCR, "RAPIRUN S. pneumoniae HS", and "BinaxNOW Streptococcus pneumoniae" were approximately the same (Table 4).

**[Table 4]**

| | Specimen sampling day | Severity of pneumonia | Sputum | Urine | Blood | | | |
|---|---|---|---|---|---|---|---|---|
| | | | Culture | Binax | PCR | RAPIRUN HS | Culture | Binax |
| F-7321 | 1st day | -* | positive | NT | <200 | (-) | NT | (-) |
| F-7322 | 2 days after | Severe | positive | NT | 6000 | (+) | NT | (+) |
| F-7308 | 4 days after | Extremely severe | negative | (+) | 40000 | (+) | (-) | (+) |
| F-7310 | 4.5 days after | Extremely severe | NT | NT | 200000 | (+) | NT | (+) |
| F-7314 | 5 days after | Extremely severe | NT | (+) | 200000 | (+) | (-) | (+) |
| F-7317 | 6 days after | Extremely severe | negative | NT | 70000 | (+) | (-) | (+) |

| | | | | | | | | |
|---|---|---|---|---|---|---|---|---|
| RAPIRUN HS: RAPIRUN S. pneumoniae HS, Binax: BinaxNOW Streptococcus pneumoniae *: Diagnosed with bronchitis | | | | | | | | |

### Example 3: Correlation of a combination with measured biochemical values with severity

(1) Pneumonia cases were classified into a group of severe or extremely severe cases and a group of others and moderate or less cases (including mild cases), and each group was further classified into a subgroup of "RAPIRUN S. pneumoniae HS" (indicated by HS in the drawings) positive cases and a subgroup of negative cases. The measured values of biochemical markers (CRP, WBC, and BUN) were plotted on the vertical axis.

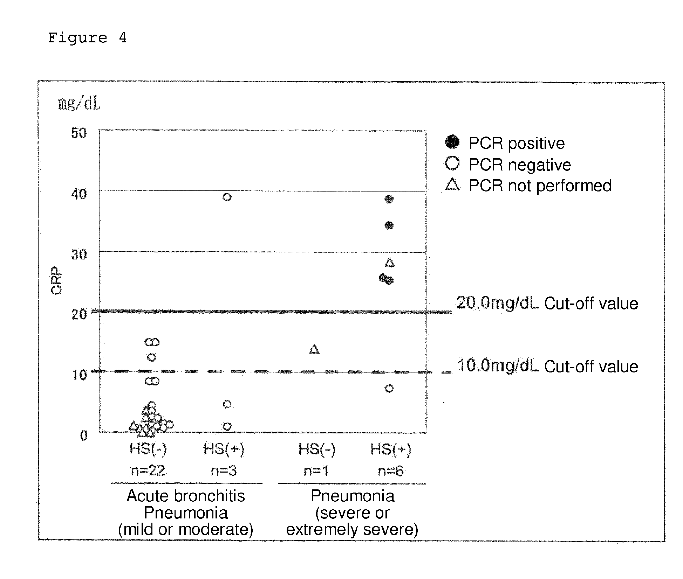

### 1) CRP (Figure 4)

In a combination of CRP and "RAPIRUN S. pneumoniae HS", S. pneumoniae PCR positive group could be extracted. It was believed that the optimal cut-off value of CRP in this case was 20 mg/dL.

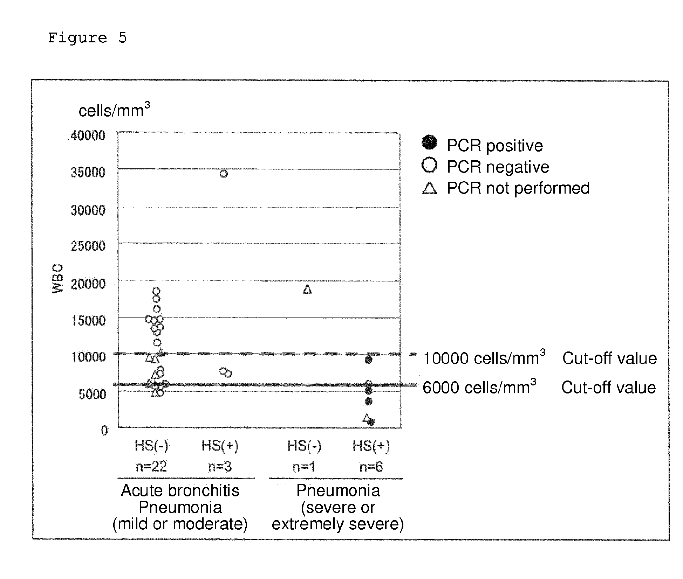

### 2) WBC (Figure 5)

In a combination of WBC and "RAPIRUN S. pneumoniae HS", PCR positive cases could be efficiently detected. It is believed that the upper limit of the distribution range in healthy adults, 10000 cells/mm³, can be employed as the cut-off value of WBC in this case.

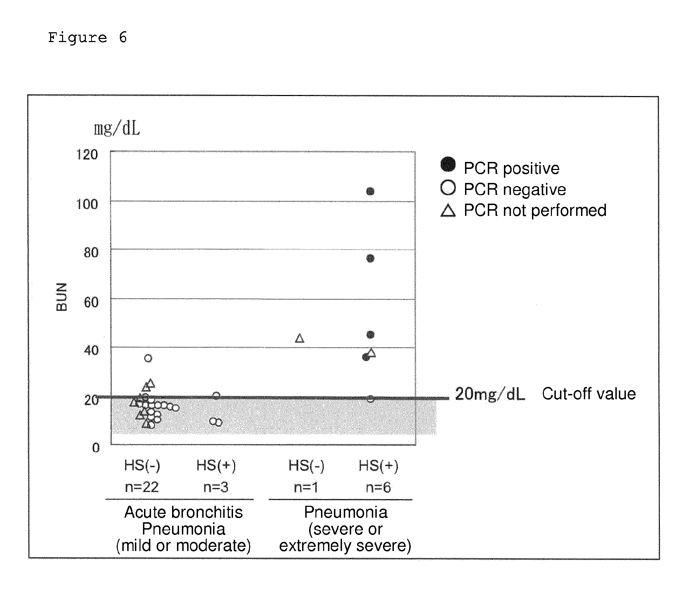

### 3) BUN (Figure 6)

In a combination of BUN and "RAPIRUN S. pneumoniae HS", PCR positive cases could be efficiently detected. It is believed that 20 mg/dL can be employed as the cut-off value of BUN in this case.

### Comparative Example

Pneumonia cases were classified into a group of severe or extremely severe cases and a group of others and moderate or less cases (including mild cases), and each group was further classified into a subgroup of "RAPIRUN S. pneumoniae HS" (indicated by HS in the drawings) positive cases and a subgroup of negative cases. The measured values of biochemical markers (neutrophil, Na, Cr, and BNP) were plotted on the vertical axis.

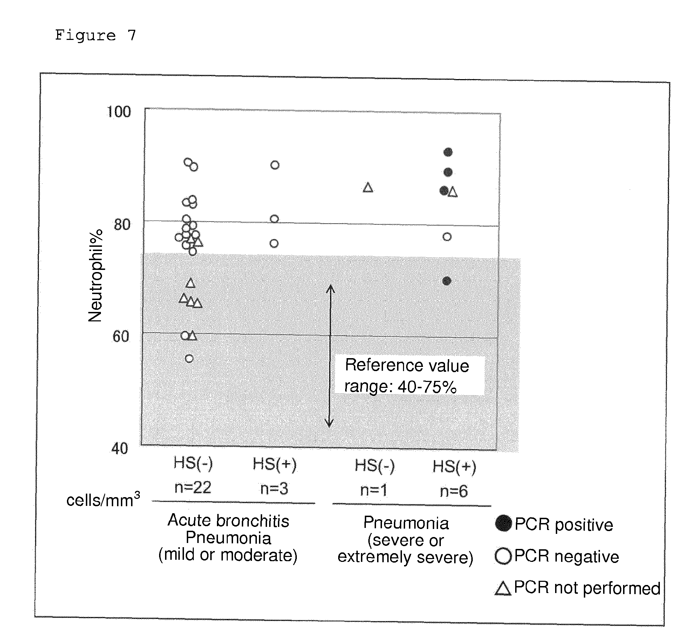

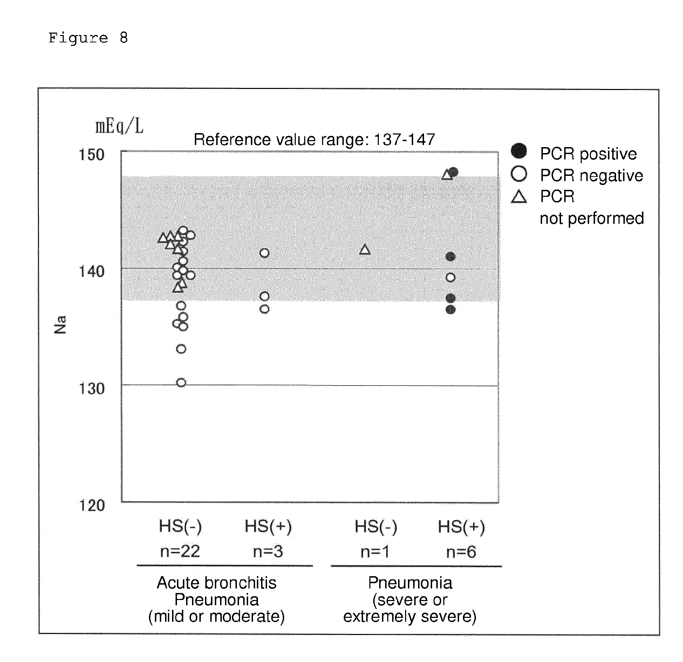

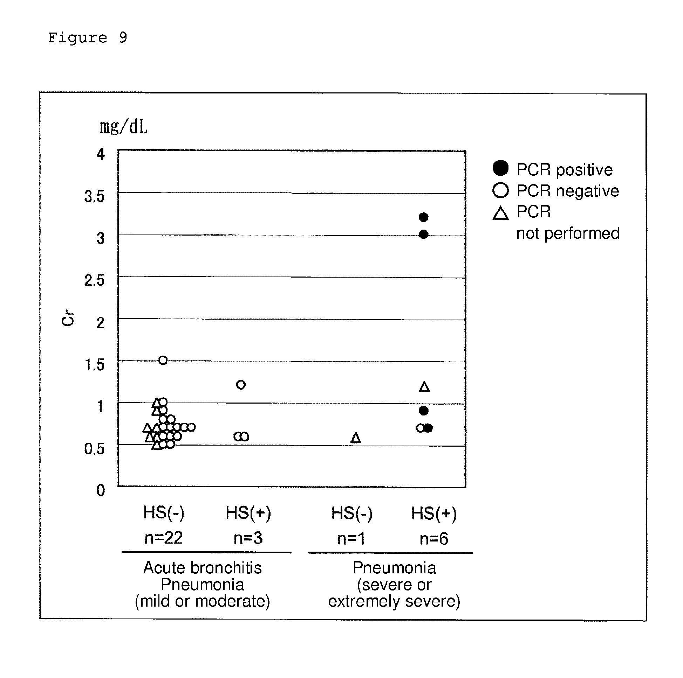

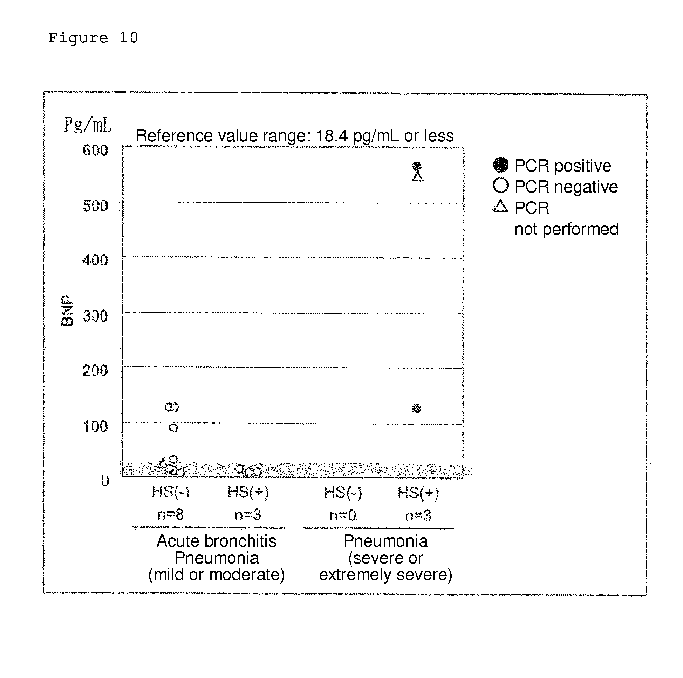

The results are shown in Figures 7 to 10. When neutrophil or Na was employed, there was no significant difference between the groups. Regarding Cr and BNP, high value cases were observed in the subgroup of RAPIRUN S. pneumoniae HS positive cases of the group of severe cases, which was limited to a part of the cases and did not show clinical significance.

### [Industrial Applicability]

In diagnosis using a biochemical marker alone, though the severity of a case suspected with pneumonia can be predicted to some extent, it is impossible to distinguish from not only S. pneumoniae but also from other infectious diseases. It has been reported that about 50% to 80% of the microorganisms causing community-acquired pneumonia in adults are bacteria and, among them, 12% to 27% of the pneumonia are pneumococcal pneumonia. Pneumococcal pneumonia is particularly apt to become severe, and discrimination of S. pneumoniae from other pathogenic microorganisms is clinically important. Furthermore, in order to prevent various pathogenic bacteria from becoming resistant to drugs, it is believed that selection of an optimal antibacterial agent by rapidly determining pathogenic bacteria is important also for shortening the duration of treatment. In the case of S. pneumoniae, the guidelines require that the first choice is a penicillin antibiotic. Accordingly, a combination of a biochemical marker and a blood pneumococcal antigen detection system can rapidly determine severe pneumococcal pneumonia, which cannot be judged by the biochemical marker alone, and can simultaneously determine severity of pneumonia, which cannot be determined by the pneumococcal antigen detection system alone, and therefore has significance for selecting an optimal antibacterial agent and judging necessity of hospital treatment.

## Claims

1. A method for determining severity of pneumococcal pneumonia, comprising determining the severity based on the presence or absence of a pneumococcal antigen in blood collected from a subject and at least one biochemical value selected from a blood C-reactive protein (CRP) level, a white blood cell (WBC) count and a blood urea nitrogen (BUN) level.

2. The determination method according to Claim 1, wherein the pneumococcal pneumonia is classified as severe or extremely severe when the pneumococcal antigen is positive and the biochemical value is in the following range:
CRP: 10 mg/dL or more,
WBC: 10,000 cells/mm³ or less, or
BUN: 20 mg/dL or more.

3. The method according to Claim 1 or 2, wherein the pneumococcal antigen is at least one selected from a C-polysaccharide antigen, a capsular antigen, and a cell membrane polysaccharide antigen.

4. The method according to any one of Claims 1 to 3, wherein pneumococcal bacteremia is simultaneously evaluated.
